# EUROPEAN PATENT APPLICATION

(11) **EP 3 698 834 A1**
(43) Date of publication of application: **26.08.2020**
(21) Application number: 19158546.2
(22) Date of filing: 21.02.2019
(51) Int. Cl.: A61M 16/00, A61M 16/01, A61M 16/20, A61M 16/06, A61M 16/10, A61M 16/12

(54) **MEDICAL GAS DELIVERY DEVICE WITH A FAST RESPONSE-TIME OXYGEN SENSOR**

(71) Applicant: L'AIR LIQUIDE, SOCIETE ANONYME POUR L'ETUDE ET L'EXPLOITATION DES PROCEDES GEORGES CLAUDE, 75007 Paris (FR)
(72) Inventor: BOULANGER, Thierry, Newark, DE Delaware DE-19702 (US)
(74) Representative: Pittis, Olivier

(57) **Abstract**

A medical gas delivery device (1) comprising an inner gas passage (100) in fluid communication with a deformable reservoir (27), and a processing unit (51). It is further provided an oxygen sensor (240) having a response time of less than 1 second arranged so as to measure the oxygen concentration in the inner gas passage (100) upstream of the deformable reservoir (27). Preferably, the oxygen sensor (240) has a response time of less than 500 msec, preferably of 300 msec or less. The oxygen sensor (240) is electrically connected to processing unit (51) for providing oxygen concentration measurement signals to said processing unit (51). The gas delivery device is useable for treating patients requiring an administration of a gas mixture such as a mixture of nitrous oxide and oxygen.

## Description

The present invention relates to a medical gas delivery device useable in various locations for treating patients, such as in hospitals, in physician or dentist offices, at home.

Some therapies require the administration of a gas mixture to patients. Thus, an equimolar (50%/50%) mixture of nitrous oxide (N₂O) and oxygen (O₂) can be used for relieving anxiety, providing light sedations or treating pain.

Generally speaking, a gas (i.e. gas or gas mixture) can be delivered to a patient either continuously or intermittently, i.e. periodically.

A gas mixture, also called "premix", is usually compressed and conditioned in a gas cylinder or any other suitable container. For security reasons, such as fire hazards, gas cylinders are usually stored in separated rooms with natural ventilation and no direct heating, including outdoor conditions.

This can be a problem with some specific compounds, such as N₂O, that can easily return into a liquid state under specific temperature and pressure conditions.

For instance, if a cylinder containing an equimolar mixture of O₂/N₂O, filled at a pressure of about 140 bars, is exposed to a temperature of -6°C or less, N₂O will liquefy in a process called lamination. This leads to potentially hypoxic mixtures as when a patient inhales such a mixture, it receives a high O₂ concentration when the cylinder is full (i.e. with little analgesic effects) and, conversely, an increasing N₂O content as the cylinders depletes.

For this reason, gas manufacturers advocate to store the gas cylinders in a heated location, for example in the hospital, at least several hours before their use. Further, the users have to periodically "shake" the cylinders, namely turn them upside down, to ensure a good mixing of the compounds thereby providing a proper mixture quality.

On can easily understand that such proceedings are not convenient, time consuming and can involve mistakes and risks for the patients, for instance confusion risks, when several cylinders are stored in a same room by different people.

Hence, being able to detect a wrong gas mixture, i.e. containing compounds not correctly mixed, is very important for lowering the risks to the patients.

It is the purpose of the present invention to provide a gas delivery device that can detect that the mixture concentration is out of a given range thereby allowing taking appropriate measures for suppressing or lowering the risk for the patients that have to inhale said gas mixture, especially a N₂O/O₂ gas mixture.

A solution according to the present invention concerns a gas delivery device comprising an inner gas passage in fluid communication with a deformable reservoir, and a processing unit, characterized in that it further comprises an oxygen sensor having a response time of less than 1 second arranged so as to measure the oxygen concentration in the inner gas passage upstream of the deformable reservoir.

Depending on the embodiment, the gas delivery device according to the present invention can comprise one or several of the following features :
- the oxygen sensor has a response time of less than 500 msec.
- the oxygen sensor has a response time of less than 400 msec, preferably of 300 msec or less.
- the oxygen sensor has a response time of less than 200 msec.
- the oxygen sensor has a response time of less than 100 msec.
- the oxygen sensor has a response time of 50 msec or less.
- the oxygen sensor is arranged in inner gas passage.
- it further comprises an air entry line fluidly branched to inner gas passage, the oxygen sensor being arranged in inner gas passage between the deformable reservoir and the air entry line.
- oxygen sensor is configured to measure the oxygen concentration in the gas flow circulating into inner gas passage, preferably after its mixing with air provided by air entry line.
- oxygen sensor is chosen among paramagnetic sensors.
- oxygen sensor is chosen among fluorescence quenching sensors.
- oxygen sensor is electrically connected to processing unit for providing oxygen concentration measurement signals to said processing unit.
- the processing unit is configured for processing the measurement signal(s) provided by the oxygen sensor, namely oxygen concentration signals, and converting it/them into oxygen concentration(s), preferably by means of a lookup table that can be memorized in memory means.
- the processing unit is configured for comparing the measured oxygen concentration(s) to an oxygen concentration range, for instance a memorized oxygen range.
- said oxygen range comprises a lower O₂ threshold and an upper O₂ threshold corresponding to minimum and maximum oxygen values, respectively.
- the oxygen range comprises oxygen values (mol.%) from 40 to 60%, preferably of between 45 and 55%, more preferably from 48% to 52%.
- for instance, the lower O₂ threshold equals 48% (mol.%) and the upper O₂ threshold equals 52% (mol.%).
- the processing unit is configured for stopping the delivery of gas, i.e. stopping the therapy, and/or for triggering an alarm system, i.e. warning the user, when said processing unit determines that the measured oxygen concentration(s) is not comprised in the oxygen concentration range, namely when the measured oxygen concentration(s) is less than the lower O₂ threshold (e.g. <48 mol.%) or more than the upper O₂ threshold (e.g. >52 mol.%).
- the processing unit is configured for triggering an alarm system providing audio and/or visual alert(s).
- it further comprises a proportional valve arranged on the inner gas passage for controlling the flowrate of gas in said inner gas passage.
- oxygen sensor is arranged downstream of proportional valve.
- the processing unit is configured for controlling the proportional valve for prohibiting any gas to pass trough said proportional valve when the measured oxygen concentration(s) is not comprised in the oxygen concentration range, preferably the processing unit are configured for closing the proportional valve.
- the alarm system is further conceived for making the user aware in case of further problem(s) affecting the device or the gas, for instance a valve or sensor failure.
- it further comprises reservoir detection means cooperating with the deformable reservoir and with the processing unit for determining a distance (D) between said reservoir detection means and said deformable reservoir, wherein the processing unit controls the proportional valve for adjusting the flowrate of gas traversing said proportional valve on the basis of said distance (D) between the reservoir detection means and the deformable reservoir.
- the distance (D) is determined at given time intervals, preferably every 100 msec or less, more preferably every 50 msec or less.
- said distance (D) is calculated using signals delivered by the reservoir detection means and processed by the processing unit.
- the reservoir detection means are or comprise a reservoir detection device or the like.
- the distance (D) depends on the quantity of gas comprised into the deformable reservoir and is comprised between i) a distance at rest (Drest) corresponding to a deformable reservoir full of gas, and ii) a given deflated distance (Dmax) corresponding to a deformable reservoir at least partially deflated, with Dmax>Drest.
- the distance (D) is measured between the reservoir detection means and the outer wall of the deformable reservoir, i.e. the peripheral surface of the deformable reservoir, preferably to a specific area located on the peripheral surface of the deformable reservoir.
- the flowrate of gas traversing the proportional valve is set by the processing unit so as to be proportional to the distance (D).
- the processing unit controls the proportional valve for increasing or for decreasing the flowrate of gas traversing the proportional valve based on the distance (D). For instance, when the distance (D) increases, then the flowrate of gas is increased too, as a distance increase means that the reservoir is at least partially deflated, and vice versa.
- the inner gas passage comprises one or several conduits or the like.
- the reservoir detection means comprise a distance sensor.
- the deformable reservoir is made of a flexible material, preferably a rubber material or the like, such as silicone rubber LSR from NuSil.
- the distance sensor is arranged on a sensor-support, such as a plate or the like.
- the distance sensor arranged in the vicinity of reservoir, typically at a distance less than 10 cm, preferably at a distance of between 0.5 and 5 cm.
- the distance sensor is a "time of flight" sensor.
- the distance sensor is a "time of flight" sensor comprising emitter means and a receiver means.
- emitter means (i.e. an emitter device) are configured for emitting a forward signal at given time intervals toward reservoir.
- emitter means are configured for emitting a forward signal at time intervals of between 1 msec and 1000 msec, preferably between 10 and 500 msec, more preferably less than 100 msec.
- emitter means are configured for emitting a forward signal preferably every 50 msec.
- receiver means (i.e. a receiver device) are configured for receiving a return signal corresponding to the forward signal that is returned after having hit the reservoir, preferably a specific area located on the outer wall of reservoir, i.e. its peripheral surface.
- the forward signal and the return signal are light signals.
- the forward signal is a light pulse.
- emitter means comprise a laser diode for emitting light signals, e.g. light pulses.
- receiver means comprise a photodiode for receiving light signals.
- the processing unit is configured for:
   a) processing the forward and return signals received from the reservoir detection means,
   b) determining a propagation time between said forward and return signals, and
   c) deducing from said propagation time, the distance (D) between the reservoir detection means and the deformable reservoir. Actually, the distance (D) reflects a degree of inflation/deflation of the deformable reservoir, which corresponds to a residual volume of gas into the reservoir, for instance reservoir full, empty or in-between.
- the processing unit comprises a (or several) microprocessor(s), preferably a microcontroller.
- the processing unit comprises a (or several) microprocessor running one or several algorithms, preferably a (or several) microcontroller(s).
- the processing unit comprises a (or several) memory(ies) for storing information, data, signal measurements....., in particular look-up tables or the like.
- it further comprises a housing, preferably made of polymer or the like.
- the inner gas passage, the deformable reservoir, the proportional valve, the processing unit and the detection means are arranged in said housing.
- the inner gas passage is fluidly connected to a source of therapeutic gas.
- the source of therapeutic gas is a gas cylinder.
- the source of therapeutic gas contains N₂O, preferably a mixture of N₂O and oxygen.
- the source of therapeutic gas contains a binary mixture N₂O/O₂ containing 50 mol.% or less of N₂O and oxygen for the rest.
- the source of therapeutic gas contains an equimolar mixture N₂O/O₂ (i.e., 50/50 mol.%).
- the inner gas passage is fluidly connected to an air entry line, preferably upstream of the flexible reservoir and/or downstream of the proportional valve, i.e. in-between.
- it further comprises a flow sensor arranged in the inner gas passage for measuring the flow of gas (i.e. flowrate) circulating into the lumen of said inner gas passage.
- the flow sensor is arranged downstream of the proportional valve for measuring the flow of gas delivered by said proportional valve.
- the flow sensor is arranged upstream of the flexible reservoir, preferably upstream of the oxygen sensor, more preferably upstream of the air entry line.
- the flow sensor is a mass flow sensor or a differential pressure sensor.
- the deformable reservoir comprises (at rest) an internal volume of about between 0.5 and 3 L.
- the deformable reservoir comprises a peripheral wall having a thickness of between about 0.10 and 0.90 mm, typically of between about 0.25 and 0.50 mm.
- a (or several) one-way valve element(s) is arranged in the inner gas passage downstream of the deformable reservoir.
- it further comprises a differential pressure sensor for measuring the pressure drop generated by said one-way valve when a flow passes through it.
- said differential pressure sensor is arranged on a by-pass conduit fluidly connected to the inner gas passage, upstream and downstream of the one-way valve.
- it further comprises a power source for providing electric current to the different components or parts of the device in need thereof for working.
- it further comprises a man-machine interface, such as a touchscreen and buttons.
- it further comprises a digital display performed by the touchscreen.
- it further comprises an on/off actuator, such as a button, a touch switch or the like for switching on or off the device.

The present invention also concerns a method for providing a respiratory gas to a patient, i.e. a human being, in need thereof comprising :
a) providing a gas delivery device according to the present invention,
b) delivering a respiratory gas to the patient's airways using said gas delivery device.

Depending on the embodiment, the method for providing a respiratory gas to a patient according to the present invention can comprise one or several of the following features :
- it further comprises providing a source of respiratory gas, preferably a gas cylinder containing the respiratory gas, especially a therapeutic gas.
- the respiratory gas contains a therapeutic gas containing one or several gaseous compounds, i.e. a gas or a gas mixture.
- the therapeutic gas contains N₂O.
- the therapeutic gas contains a mixture of N₂O and oxygen (O₂).
- the therapeutic gas contains a mixture N₂O/O₂ containing 50 mol.% or less of N₂O and oxygen (O₂) for the rest.
- the therapeutic gas contains an equimolar mixture N₂O/O₂ (50/50 mol.%).
- fluidly connecting the source of respiratory gas to the gas delivery device, preferably by means of a first flexible hose or the like.
- further fluidly connecting the gas delivery device to the patient's airways, preferably by means of a second flexible hose or the like.
- the respiratory gas is delivered to the patient by means of a respiratory interface, such as a respiratory mask or the like, preferably an oro-nasal mask.

The present invention will be explained in more details in the following illustrative description of an embodiment of a gas delivery device according to the present invention, which is made in references to the accompanying drawings among them :
- Fig. 1 is a schematic representation of an embodiment of a gas delivery device according to the present invention, and
- Fig. 2 shows the internal architecture of the gas delivery device of Figure 1.

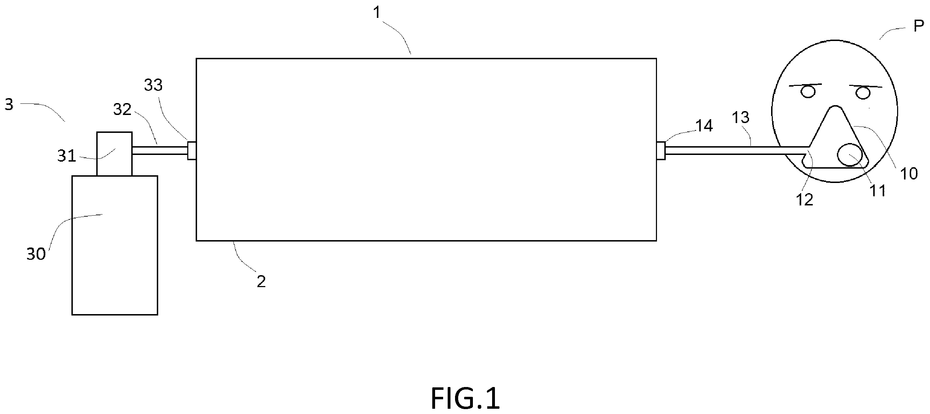

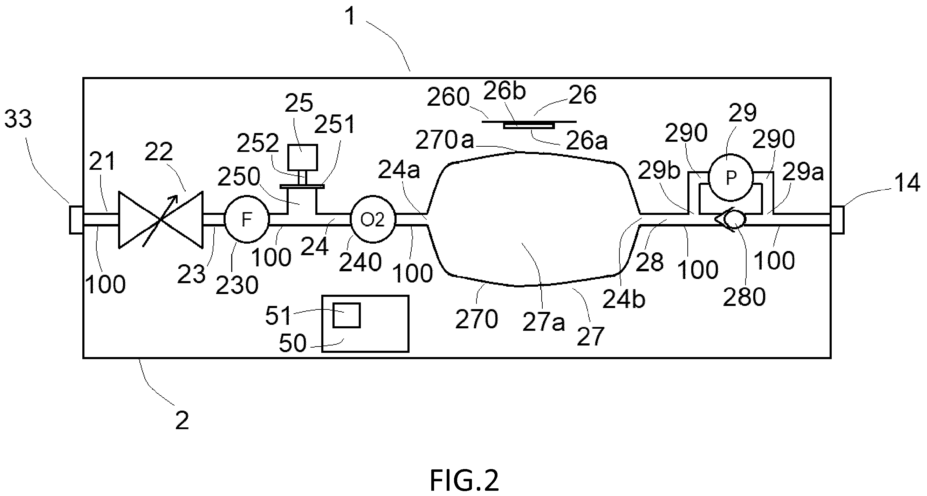

Figure 1 is schematic representation of an embodiment of a gas delivery device 1 according to the present invention. The gas delivery device 1 comprises a housing 2 or casing, for instance made of polymer, comprising components of the gas delivery device 1, as detailed below in reference to Figure 2.

A gas source 3, such as a gas cylinder 30 equipped with a valve 31, provides a respiratory gas, i.e. a gas or gas mixture, to the gas delivery device 1 by means of a gas line 32, such as a flexible hose or the like, that is fluidly connected to an inlet port 33 of the gas delivery device 1. The respiratory gas circulates into the gas delivery device 1, as detailed below, and is subsequently conveyed to a patient P by means of a flexible tube 13, i.e. a conduit, a hose or the like, that is fluidly connected to an outlet port 14 of the gas delivery device 1. The gas is administered to the patient P by means of a respiratory interface 10, such as a respiratory mask, that is fed by the flexible tube 13.

In Figure 1, the respiratory interface 10 is an oro-nasal mask covering the patient's mouth and nose. Other respiratory interfaces may also be suitable. The oro-nasal mask 10 exhibits an exhalation port 11 and inhalation port 12. The inhalation port 12 is fluidly connected to flexible tube 13 that conveys the gas to be inhaled from the outlet port 14 of the device 1 to the patient. The exhalation valve 11 is preferably a one-way valve that vents the CO₂-enriched gas exhaled by the patient P to the atmosphere, and that further prevents any backflow of ambient air coming from the atmosphere, when the patient P inhales respiratory gas, i.e. during inhalation phases. The one-way valve comprises a flexible silicone disk laying on a perforated surface that allows gas passing through unidirectionally, i.e. only in one way, for instance, the layout "membrane/perforated surface" of the valve sold by QOSINA under reference #97351.

The gas source 3 contains a pressurized gas, for instance an equimolar mixture (50%/50% ; mol.%) of N₂O and O₂ at a maximal pressure of between 170 and 250 bars abs (when full of compressed gas). Valve 31 is preferably an integrated pressure-regulator valve 31 delivering the gas into hose 32 at a given reduced pressure, for instance a reduced pressure of 4 bar abs. Valve 31 is preferably protected by a rigid cap arranged around it (not shown).

Figure 2 shows an embodiment of the different elements arranged into the housing 2 of the gas delivery device 1 according to the present invention, i.e. of the internal architecture of the gas delivery device 1 of Figure 1, in particular the location of the oxygen sensor 240 used for measuring the oxygen concentration in the gas flow, in the inner gas passage 100 upstream of the deformable reservoir 27, as explained hereafter.

It comprises an electronic board 50 comprising a processing unit 51 including a (or several) microcontroller running an (or several) algorithm(s), which recovers and processes information, data and/or measurements provided by different actuators, sensors or the like.

An inner gas passage 100 is arranged in housing 2 between inlet port 33 and outlet port 14 so as to convey gas from inlet port 33 to outlet port 14. The inner gas passage 100 comprises several successive passage sections 21, 23, 24, 28.

The gas inlet port 33 carried by the rigid housing 2 of the gas delivery device 1 is in fluid communication with the upstream section 21 of inner gas passage 100. A proportional valve 22 is arranged on inner gas passage 100, preferably in the upstream part of inner gas passage 100 between first and second sections 21, 23. The proportional valve 22 is controlled by the microcontroller of the processing unit 51 for adjusting the gas flow circulating in the lumen of the inner gas passage 100 as detailed hereafter. Different types of proportional valves 22 can be used, such as proportional valves referenced IMI FAS FLATPROP or FESTO VEMR.

The gas flow passing through and exiting proportional valve 22 is recovered and conveyed by inner gas passage 100, namely the second section 23.

A flow sensor 230 is arranged in inner gas passage 100 for measuring the flow (i.e. flowrate) of the gas provided by proportional valve 22. Flow sensor 230 can be a mass flow sensor or a differential pressure sensor, preferably a differential pressure sensor. Flow sensor 230 is electrically connected to processing unit 51. Flow sensor 230 delivers a flow signal that is further processed by processing unit 51, namely the microcontroller. Preferably, a volumetric flow is obtained after conversion of the flow signal using a specific look-up table that is memorized in a memory cooperating with the processing unit 51.

Flow sensor 230 can also be used for detecting any default fault of proportional valve 22 or for determining the quantity of gas (i.e. volume) delivered by gas source 3.

Further, the gas delivery device 1 according to the present invention also comprises an air entry line 250, such as a conduit or the like, fluidly connected to the inner gas passage 100, downstream of the flow sensor 230, i.e. fluidly branched to third section 24. Air entry line 250 provides ambient air that mixes with the therapeutic gas traveling in the lumen of inner gas passage 100, preferably a N₂O/O₂ gas mixture.

According to the invention, an oxygen sensor 240 is arranged in inner gas passage 100, downstream of the air entry line 250. Oxygen sensor 240 is arranged and configured so as to measure the oxygen concentration in the gas flow circulating into inner gas passage 100 after its mixing with air provided by air entry line 250, i.e. in third section 24. In the frame of the present invention, "arranged in inner gas passage 100" means that the active sensing portion (e.g. sensitive to the oxygen concentration) of oxygen sensor 240 is in direct contact with the gas circulating into inner gas passage 100 and that consequently oxygen sensor 240 is mechanically coupled to said inner passage 100. As the integration process of oxygen sensors into gas conduits is well known in the art, such assemblage is not represented.

Oxygen sensor 240 has a fast response time, for example 1s or less, such as less than 400 msec, preferably 200 msec or less, more preferably or 50 msec or less.

An oxygen sensor 240 with a fast response time is preferred over traditional oxygen sensing technologies, such as electrochemical cells. Indeed, electrochemical cells typically have a response time of about 30s. This means that about 30s are required to detect any "abnormality" in the gaseous mixture, such as a lack of oxygen or an excess of N₂O for instance. In extreme cases, i.e. hypoxic gas mixture, a patient inhaling the gas mixture would be put at risk.

An oxygen sensor 240 with a fast response time, as per the present invention, will prevent such situation to occur.

In some embodiments, paramagnetic sensors are useable, such as the sensor called Paracube Micro sold by Hummingbird Technologies.

In other embodiments, fluorescence quenching sensors are also useable, such as oxygen sensor from S4MS or another fluorescence quenching sensor.

Oxygen sensor 240 is also electrically connected to processing unit 51 and providing measurement signals to processing unit 51. Processing unit 51 processes said measurement signals and converts them into oxygen concentrations via a lookup table.

As discussed, processing unit 51 expects said oxygen concentration to be close to 50%. It can be determined that said processing unit 51 compares the measured oxygen concentrations to a lower threshold, for example 48% and an upper threshold, for example 52%. If the measured oxygen concentration does not fall in the 48%-52% range, processing unit 51 has the ability to stop the therapy and warn the user through audible and/or visual signals.

The entering of air into air entry line 250 is controlled by a valve element 251, such as a disc shaped membrane, that normally prohibits air entering into air entry line 250. Valve element 251 cooperates with an actuator 25 comprising an acting part 252, like a stem or the like, mechanically coupled to the valve element 251. Actuator 25 is controlled by processing unit 51 and acts on the valve element 251, via acting part 252, for proportionally allowing or prohibiting the entering of air into air entry line 250. For instance, valve element 251 can be moved up for progressively allowing air entering into air entry line 250 by an air inlet (i.e. orifice or the like) or down for progressively prohibiting or stopping air entering into air entry line 250. Actuator 25 can be a linear actuator, for instance an actuator commercialized under reference 26DAM by Portescap.

The inner gas passage 100 of the gas delivery device 1 according to the present invention afterwards provides the gas flow to a deformable reservoir 27, in particular a flexible reservoir, arranged downstream of air entry line 250 and oxygen sensor 240, and in fluidic connection with inner gas passage 100, namely with third section 24.

Deformable reservoir 27 comprises a flexible peripheral wall 270 delimiting an internal volume 27a for the gas, thereby forming a "deformable bag" for the gas. At rest, deformable reservoir 27 exhibits an internal volume 27a of about between 0.5 and 3L for instance. It is further provided a specific area 270a located on the outer wall 270 of reservoir 27.

The flow of gas enters into the internal volume 27a of the deformable reservoir 27 through a reservoir inlet orifice 24a in fluid communication with inner passage 100.

Preferably, the properties of the deformable reservoir 27 are such that it is highly deformable. For instance, its peripheral wall 270 has a thickness of between about 0.25 and 0.5 mm and is made of a flexible, biocompatible silicone rubber, such as LSR series commercialized by NuSil.

The distance sensor 26 is arranged in housing 2 in the vicinity of reservoir 27. Distance sensor 26 is securely attached to a sensor support 260, such as a plate or the like, that is arranged and secured into housing 2. Distance sensor 26 is preferably a "time of flight" sensor that comprises emitter means 26a, i.e. a signal emitter, such as a laser diode, and receiver means 26b, i.e. a signal receiver, such as a photodiode.

At frequent intervals, for instance every 50 msec, emitter means 26a send a forward signal, typically a light pulse, toward the reservoir 27, which reaches preferably the specific area 270a located on the outer wall 270 of reservoir 27. At least a fraction of said emitted signal is bouncing back (i.e. return or back signal) and hits the photodiode 26b. The time (i.e. duration) between emission and reception of the forward and return signals is proportional to the distance D between the sensor 26 and area 270a. The smaller the time, the closer area 270a from sensor 26 and conversely, the longer the time, the farer the area 270a from sensor 26.

Exploiting this information and processing it via a specific look-up table, the processing unit 51, i.e. microcontroller, can determine the distance D between the sensor 26 and the area 270a of said reservoir 27 that reflects or corresponds to the degree of inflation/deflation of flexible reservoir 27.

Furthermore, as shown in Figure 2, the gas leaves the internal volume 27a of reservoir 27 by a reservoir outlet orifice 24b that is fluidly connected to a downstream section 28 of inner gas passage 100 that terminates at outlet port 14.

A (or several) one-way valve element 280 is arranged in the inner gas passage 100, downstream of reservoir 27, namely between reservoir outlet orifice 24b and outlet port 14 of housing 2, for preventing any backflow of gas. Thus, gas exhaled by patient P are vented only through exhalation port 11 of mask 10 and cannot return into reservoir 27. One-way valve 280 is preferably designed such that a very low pressure drop (i.e. < 0.2 mbar) is generated across it, when a flow of gas travels through it. In another embodiment, several one-way valve elements 280 can also be used in lieu of only one, for example 3 to 5 arranged in parallel (not shown).

It is further provided a differential pressure sensor 29 for measuring the pressure drop generated by said one-way valve 280 when a flow is passing through it. Differential pressure sensor 29 is arranged on a by-pass conduit 290 fluidly connected to the inner gas passage 100, upstream and downstream ('U'-shape) of said one-way valve 280 for allowing a measurement of the pressures in inner gas passage 100, at two locations 29a, 29b, namely upstream 29b and downstream 29a of one-way valve 280. Pressure signals measured by the differential pressure sensor 29 are sent and then processed by the microcontroller of the processing unit 51. Typically, pressure signals are converted into a flow using a specific look-up table corresponding to the pressure-flow relationship of one-way valve 280. For instance, the differential pressure sensor "SDP3X series" from Sensirion can be used.

A power source (not shown) is preferably arranged in housing 2, such as a rechargeable battery, for delivering electric current (i.e. power) to all the components working with electric current, such as sensors, processing unit, controlled-valves, reservoir detection means, man-machine interface, digital display....

A calibration of oxygen sensor 240 can be operated as the gas travelling into passage 100 is ambient air (i.e. 21 % O₂). Once, the oxygen sensor 240 is stabilized, e.g. has been in contact with ambient air for enough time, the processing unit 51 can perform a calibration of said oxygen sensor 240.

This calibration point helps determining a new look-up table that takes into account any drift having occurred in said oxygen sensor 240 to guarantee an appropriate accuracy of the oxygen concentration measurement.

This ability to perform a calibration at ambient air is critical. Indeed, any sensor, and especially oxygen sensors, drift over time. Without regular calibration, oxygen sensor 240 will progressively loose accuracy, meaning that processing unit 51 may detect an oxygen concentration out of the 48%-52% range whereas the real oxygen concentration is still 50%. This would make the system strictly unusable.

The possibility of performing a calibration of oxygen sensor 240 through its exposition to the equimolar O₂/ N₂O mixture must obviously be discarded as it makes no sense to calibrate a sensor against a metric that it is supposed to measure. For example if the real oxygen concentration of the mixture is 30%, a calibration at the "50%" point of oxygen sensor 240 would create a huge inaccuracy, making the system likewise strictly unusable.

Hence, a calibration at ambient air (i.e. 21% O₂) is the only viable and most preferred option to ensure an appropriate measurement accuracy.

Generally speaking, a gas delivery device 1 according to the present invention can be used for providing a respiratory gas, especially a therapeutic gas, preferably containing N₂O and oxygen, to a patient in need thereof.

## Claims

1. Gas delivery device (1) comprising an inner gas passage (100) in fluid communication with a deformable reservoir (27), and a processing unit (51), **characterized in that** it further comprises an oxygen sensor (240) having a response time of less than 1 second arranged so as to measure the oxygen concentration in the inner gas passage (100) upstream of the deformable reservoir (27).

2. Gas delivery device according to the preceding Claim, **characterized in that** the oxygen sensor (240) has a response time of less than 500 msec, preferably of 300 msec or less.

3. Gas delivery device according to any one of the preceding Claims, **characterized in that** the oxygen sensor (240) has a response time of less than 200 msec.

4. Gas delivery device according to any one of the preceding Claims, **characterized in that** the oxygen sensor (240) has a response time of less than 100 msec, preferably of 50 msec or less.

5. Gas delivery device according to any one of the preceding Claims, **characterized in that** the oxygen sensor (240) is arranged in inner gas passage (100).

6. Gas delivery device according to any one of the preceding Claims, **characterized in that** it further comprises an air entry line (250) fluidly branched to inner gas passage (100), the oxygen sensor (240) being arranged in inner gas passage (100) between the deformable reservoir (27) and the air entry line (250).

7. Gas delivery device according to any one of the preceding Claims, **characterized in that** oxygen sensor (240) is configured to measure the oxygen concentration in the gas flow circulating into inner gas passage (100), preferably after its mixing with air provided by air entry line (250).

8. Gas delivery device according to any one of the preceding Claims, **characterized in that** oxygen sensor (240) is chosen among paramagnetic sensors and fluorescence quenching sensors.

9. Gas delivery device according to any one of the preceding Claims, **characterized in that** oxygen sensor (240) is electrically connected to processing unit (51) for providing oxygen concentration measurement signals to said processing unit (51).

10. Gas delivery device according to any one of the preceding Claims, **characterized in that** it further comprises a proportional valve (22) arranged on the inner gas passage (100) for controlling the flowrate of gas in said inner gas passage (100).

11. Gas delivery device according to any one of the preceding Claims, **characterized in that** oxygen sensor (240) is arranged downstream of proportional valve (22).

12. Gas delivery device according to any one of the preceding Claims, **characterized in that** the processing unit (51) comprises a microprocessor, preferably a microcontroller.

13. Gas delivery device to any one of the preceding Claims, **characterized in that** the processing unit is configured for processing measurement signal(s) provided by the oxygen sensor and converting said measurement signal(s) into oxygen concentration(s).

14. Gas delivery device according to any one of the preceding Claims, **characterized in that** the processing unit is configured for comparing the measured oxygen concentration(s) to an oxygen concentration range.

15. Gas delivery device according to any one of the preceding Claims, **characterized in that** the processing unit is configured for stopping the delivery of gas, and/or for triggering an alarm system, when said processing unit determines that the measured oxygen concentration(s) is not comprised in the oxygen concentration range.
